# EUROPÄISCHE PATENTSCHRIFT

(11) **EP 0 800 508 B1**
(45) Veröffentlichungstag und Bekanntmachung des Hinweises auf die Patenterteilung: **05.04.2000**
(21) Anmeldenummer: 95941718.9
(22) Anmeldetag: 16.12.1995
(51) Int. Cl.: C07C 253/30, C07C 255/24

(54) **VERFAHREN ZUR HERSTELLUNG VON ALIPHATISCHEN ALPHA,OMEGA-AMINONITRILEN**
PROCESS FOR PREPARING ALIPHATIC ALPHA,OMEGA-AMINONITRILES
PROCEDE DE PREPARATION D'AMINONITRILES-ALPHA,OMEGA ALIPHATIQUES

(30) Priorität: 27.12.1994 DE 4446893
(43) Veröffentlichungstag der Anmeldung: 15.10.1997
(73) Patentinhaber: BASF AKTIENGESELLSCHAFT, 67056 Ludwigshafen (DE)
(72) Erfinder: MELDER, Johann-Peter, D-67141 Neuhofen (DE); SCHNURR, Werner, D-67273 Herxheim (DE); FLICK, Klemens, D-76863 Herxheim (DE); EBEL, Klaus, D-68623 Lampertheim (DE); WITZEL, Tom, D-67069 Ludwigshafen (DE); FISCHER, Rolf, D-69121 Heidelberg (DE); HARDER, Wolfgang, D-69469 Weinheim (DE); REHFINGER, Alwin, D-67112 Mutterstadt (DE)
(86) Internationale Anmeldenummer: EP9504985
(87) Internationale Veröffentlichungsnummer: WO9620166

(56) Entgegenhaltungen:
- EP-A- 0 077 911
- EP-A- 0 161 419
- WO-A-92/21650
- WO-A-93/12073
- WO-A-93/16034
- DE-A- 4 235 466
- DE-C- 848 654
- US-A- 2 257 814

## Beschreibung

Die vorliegende Erfindung betrifft ein verbessertes Verfahren zur Herstellung von aliphatischen alpha,omega-Aminonitrilen durch partielle Hydrierung von aliphatischen alpha,omega-Dinitrilen bei erhöhter Temperatur und erhöhtem Druck in Gegenwart eines Lösungsmittels und eines Katalysators.

Die WO 92/21650 beschreibt die partielle Hydrierung von Adipodinitril zu 6-Aminocapronitril in Gegenwart eines Raney-Nickel-Katalysators und Ammoniak als Lösungsmittel mit einer Ausbeute von 60% bei einem Umsatz von 70%. Als Nebenprodukt entsteht 9% Hexamethylendiamin. Nachteilig an diesem Verfahren ist die geringe Standzeit des Katalysators.

In der US 2,257,814 und in der US 2,208,598 werden ebenfalls Herstellverfahren von 6-Aminocapronitril ausgehend von Adipodinitril beschrieben, wobei als Katalysatoren Raney-Cobalt, Eisen-, Nickel- und Cobalt- Katalysatoren auf verschiedenen Trägern eingesetzt werden. Nachteilig an diesen Verfahren sind die mit 50 bis 60% für technische Anwendungen zu niedrigen Selektivitäten.

Nach dem Verfahren der WO 93/16034 kann man die Ausbeute an Aminocapronitril dadurch steigern, daß man Adiponitril in Gegenwart von Raney-Nickel, einer Base wie Natrium-, Kalium-, Lithium- oder Ammoniumhydroxid und einer Übergangsmetall-Komplexverbindung, mit beispielsweise Eisen, Cobalt, Chrom oder Wolfram als Übergangsmetalle, und eines Lösungsmittels hydriert. Nach diesem Verfahren werden bei Umsätzen im Bereich von 45 bis 60% quantitative Ausbeuten an Aminocapronitril beschrieben. Nachteilig an diesem Verfahren ist die Aufarbeitung der zumeist toxischen Übergangsmetall-Komplexverbindungen aus den erhaltenen Reaktionsgemischen.

In der EP-A 161,419 wird die partielle Hydrierung von Adipodinitril unter Verwendung eines Rhodium-haltigen Katalsators auf einem Magnesiumoxid-Träger beschrieben. Bei einem Umsatz von 70% wird eine Selektivität von 94% erreicht. Nachteilig ist die aufwendige Herstellmethode der Rh/MgO-Katalysatoren (s. J. of Cat. 112 (1988), S. 145-156).

Die DE-A 4,235,466 beschreibt die Festbetthydrierung von Adiponitril zu 6-Aminocapronitril an nach einer speziellen Methode aus Eisenerz hergestellten Eisenschwamm-Katalysatoren (Vollkontakt), die nachträglich mit Cobalt, Titan, Mangan, Chrom, Molybdän, Ruthenium oder Iridium dotiert wurden. Aufgrund der geringen Oberfläche (0,8 m²/g) zeigen diese Katalysatoren in der Regel erst bei hohen Drücken und hohen Temperaturen eine brauchbare Aktivität. Ein weiterer Nachteil dieses Verfahrens ist der rasche Aktivitätsverlust: trotz Reduktion der Adiponitril- und Wasserstoff-Belastung, was üblicherweise zu einer Umsatzerhöhung führt, ging gemäß Beispiel 7 der Umsatz innerhalb von 24 h um 5 % zurück.

Die DE-A 848,654 beschreibt die kontinuierliche Festbetthydrierung von Adipodinitril an Palladium auf Kieselgel sowie an Metallen der achten Gruppe des Periodensystems, wobei diese Metalle bevorzugt als Spinelle eingesetzt werden. Wesentlicher Nachteil dieser Katalysatoren ist deren unbefriedigende Standzeit.

Aufgabe der vorliegenden Erfindung war daher die Bereitstellung eines verbessertes Verfahrens zur Herstellung von aliphatischen alpha,omega-Aminonitrilen durch partielle Hydrierung von Adipodintril, das die zuvor genannten Nachteile nicht aufweist, insbesondere sollte ein Verfahren gefunden werden, indem die verwendeten Katalysatoren eine längere Standzeit im Vergleich zu denjenigen aus dem Stand der Technik aufweisen.

Demgemäß wurde ein Verfahren zur Herstellung von aliphatischen alpha,omega-Aminonitrilen durch partielle Hydrierung von aliphatischen alpha,omega-Dinitrilen bei erhöhter Temperatur und erhöhtem Druck in Gegenwart eines Lösungsmittels und eines Katalysators gefunden, indem man einen Katalysator verwendet, der
(a) mindestens eine Verbindung auf der Basis eines Metalles, ausgewählt aus der Gruppe, bestehend aus Nickel, Cobalt, Eisen, Ruthenium und Rhodium, enthält und
(b) von 0,01 bis 25, vorzugsweise von 0,1 bis 5 Gew.-%, bezogen auf (a), mindestens eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut, Silicium, Titan, Zirkonium und Seltenerdmetalle, sowie
(c) von 0 bis 5, vorzugsweise von 0,1 bis 3 Gew.-%, bezogen auf (a), mindestens einer Verbindung auf der Basis eines Alkalimetalles oder eines Erdalkalimetalles, enthält,
mit der Maßgabe, daß die Komponente (a) nicht auf der Basis von Eisen oder Eisen und einem der Metalle, ausgewählt aus der Gruppe, bestehend aus Cobalt, Ruthenium und Rhodium, besteht, wenn (b) ein Promotor auf der Basis eines Metalles, ausgewählt aus der Gruppe, bestehend aus Titan, Mangan, Chrom und Molybdän, ist sowie mit der weiteren Maßgabe, daß, wenn als Komponente (a) eine Verbindung auf der Basis von nur Ruthenium oder Ruthenium und Rhodium oder Nickel und Rhodium gewählt wird, der Promotor (b) gewünschtenfalls entfallen kann.

Bevorzugte Katalysatoren sind solche, in denen die Komponente (a) mindestens eine Verbindung auf der Basis eines Metalles, ausgewählt aus der Gruppe aus Nickel, Cobalt und Eisen, in einer Menge im Bereich von 10 bis 95 Gew.-% sowie Ruthenium und/oder Rhodium in einer Menge im Bereich von 0,1 bis 5 Gew.-%, jeweils bezogen auf die Summe der Komponenten (a) bis (c), enthält,
die Komponente (b) mindestens einen Promotor auf der Basis eines Metalles, ausgewählt aus der Gruppe bestehend aus Silber, Kupfer, Mangan, Rhenium, Blei, Aluminium und Phosphor, in einer Menge im Bereich von 0,1 bis 5 Gew.-%, bezogen auf (a), enthält, und
die Komponente (c) mindestens eine Verbindung auf der Basis der Alkalimetalle und Erdalkalimetalle, ausgewählt aus der Gruppe bestehend aus Lithium, Natrium, Kalium, Cäsium, Magnesium und Calcium, in einer Menge im Bereich von 0,1 bis 5 Gew.-% enthält.

Besonders bevorzugte Katalysatoren sind:
Katalysator A, enthaltend 90 Gew.-% Cobaltoxid (CoO), 5 Gew.-% Manganoxid (Mn₂O₃), 3 Gew.-% Phosphorpentoxid und 2 Gew.-% Natriumoxid (Na₂O), und
Katalysator B, enthaltend 20 Gew.-% Nickeloxid (NiO), 67,42 Gew.-% Siliciumdioxid (SiO₂), 3,7 Gew.-% Aluminiumoxid (Al₂O₃), 0,8 Gew.-% Eisenoxid (Fe₂O₃), 0,76 Gew.-% Magnesiumoxid (MgO), 1,92 Gew.-% Calciumoxid (CaO), 3,4 Gew.-% Natriumoxid (Na₂O) sowie 2,0 Gew.-% Kaliumoxid (K₂O).

Bei den erfindungsgemäß einsetzbaren Katalysatoren kann es sich um Voll- oder Trägerkatalysatoren handeln. Als Trägermaterialien kommen beispielsweise poröse Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen davon in Betracht.

Die Herstellung erfolgt in der Regel derart, daß man Vorläufer der Komponenten (a) zusammen mit Vorläufern der Promotoren (Komponenten (b) und gewünschtenfalls mit Vorläufern der Spurenkomponenten (c) in Gegenwart oder Abwesenheit von Trägermaterialien (je nachdem welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a), (b) und gewünschtenfalls (c) tränkt, wobei man die einzelnen Komponenten gleichzeitig oder nacheinander zugeben kann, oder indem man die Komponenten (a), (b) und gewünschtenfalls (c) auf den Träger nach an sich bekannten Methoden aufsprüht.

Als Vorläufer der Komponenten (a) kommen in der Regel gut wasserlösliche Salze der zuvor genannten Metalle wie Nitrate, Chloride, Acetate, Formiate und Sulfate in Betracht, vorzugsweise Nitrate.

Als Vorläufer der Komponenten (b) kommen in der Regel gut wasserlösliche Salze oder Komplexsalze der zuvor genannten Metalle wie Nitrate, Chloride, Acetate, Formiate und Sulfate sowie insbesondere Hexachloroplatinat in Betracht, vorzugsweise Nitrate und Hexachloroplatinat.

Als Vorläufer der Komponenten (c) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wäßrigen Lösungen, wahlweise durch Zugabe von Fällungsreagenzien, durch Änderung des pH-Wertes oder durch Änderung der Temperatur.

Üblicherweise trocknet man die so erhaltene Katalysatorvormasse im allgemeinen bei Temperaturen im Bereich von 80 bis 150, vorzugsweise von 80 bis 120°C vor.

Das Calcinieren nimmt man üblicherweise bei Temperaturen im Bereich von 150 bis 500, wobei in Einzelfällen auch Temperaturen bis 1000°C verwendet werden können, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 80 bis 250, vorzugsweise von 80 bis 180°C bei Katalysatoren auf der Basis von Ruthenium oder Rhodium als Komponente (a), oder im Bereich von 200 bis 500, vorzugsweise von 250 bis 400°C bei Katalysatoren auf der Basis eines der Metalle ausgewählt aus der Gruppe aus Nickel, Cobalt und Eisen als Komponente (a) 2 bis 24 h einer Wasserstoff-Atmosphäre oder einer Gasmischung, enthaltend Wasserstoff und ein Inertgas wie Stickstoff, aussetzt. Die Katalysatorbelastung beträgt hierbei bevorzugt 200 l pro l Katalysator.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 35°C mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 180 bis 500, vorzugsweise von 200 bis 350°C in einer Wasserstoff-haltigen Atmosphäre vor.

Die Katalysatoren können als Festbettkatalysatoren in Sumpf- oder Rieselfahrweise oder als Suspensionskatalysatoren eingesetzt werden.

In einer bevorzugten Ausführungsform setzt man Eisenkatalysatoren ein, deren Oberflächen durch Zugabe von Oxiden wie Aluminiumoxid stabilisiert sind. Üblicherweise werden diese Zusätze durch Zusammenschmelzen von Magnetit mit den oxidischen Zusätzen hergestellt, bevorzugt analog zu der in A.B. Stiles "Catalyst Manufacture" (1995), S. 167-168 beschriebenen Methode (siehe auch B.E. Leach, "Applied Industrial Catalysts, Vol. 3 (1984) S. 123, wo weitere Ammoniak-Synthesekatalysatoren beschrieben sind). Bevorzugte Zusätze sind Aluminiumoxid, Kaliumoxid und Calciumoxid.

Besonders bevorzugte Katalysatoren weisen eine BET-Oberfläche von größer als 5 m²/g, ganz besonders bevorzugt von 5 bis 20, insbesondere 15 bis 20 m²/g (von frisch reduzierten und mit Aluminiumoxid promotierten Eisenkatalysatoren), auf (siehe M.V. Twigg "Catalyst handbook", 2nd edition (1989) Frome, England, S. 397).

In einer weiteren bevorzugten Ausführungsform werden Eisenkatalysatoren bei Temperaturen von nicht größer als 500°C mit Wasserstoff reduziert und weisen üblicherweise einen Kohlenstoffgehalt von nicht größer als 0,4 Gew.-% auf.

Als Ausgangsstoffe im erfindungsgemäßen Verfahren werden aliphatische alpha,omega-Dinitrile der allgemeinen Formel I

NC-(CH₂)ₙ-CN I

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Suberonitril"), ganz besonders bevorzugt Adiponitril.

Nach dem erfindungsgemäßen Verfahren werden die vorstehend beschriebenen Dinitrile I in Gegenwart eines Lösungsmittels unter Verwendung eines Katalysators partiell zu alpha,omega-Aminonitrilen der allgemeinen Formel II

NC-(CH₂)ₙ-CH₂-NH₂ II

hydriert, wobei n die vorstehend genannte Bedeutung hat. Besonders bevorzugte Aminonitrile II sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansäurenitril, ganz besonders bevorzugt 6-Aminocapronitril.

Führt man die Umsetzung in einer Suspension durch, wählt man üblicherweise Temperaturen im Bereich von 40 bis 150, vorzugsweise von 50 bis 100, besonders vorzugsweise von 60 bis 90°C; den Druck wählt man im allgemeinen im Bereich von 2 bis 20, vorzugsweise von 3 bis 10, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise beim Einsatz von Adiponitril im Bereich von 50 bis 275, vorzugsweise von 70 bis 200 min.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90, vorzugsweise von 30 bis 80, besonders vorzugsweise von 40 bis 70 Gew.-%, bezogen auf die Summe von Dinitril und Lösungsmittel.

Die Menge an Katalysator wählt man im allgemeinen so, daß die Katalysator-Menge im Bereich von 1 bis 50, bevorzugt von 5 bis 20 Gew.-%, bezogen auf die eingesetzte Menge an Dinitril, beträgt.

Die Suspensionshydrierung kann man diskontinuierlich oder, bevorzugt kontinuierlich, in der Regel in der Flüssigphase durchführen.

Man kann die partielle Hydrierung auch diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 30 bis 90°C und einen Druck in der Regel im Bereich von 2 bis 30, vorzugsweise von 3 bis 20 MPa wählt. Erfindungsgemäß führt man die partielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak durch. In einer bevorzugten Ausführungsform wählt man einen Gehalt an Ammoniak im Bereich von 1 bis 10, bevorzugt von 2 bis 6 g pro g Adipodinitril. Bevorzugt wählt man dabei eine Katalysatorbelastung im Bereich von 0,1 bis 2,0, vorzugsweise von 0,3 bis 1,0 kg Adipodinitril/l*h. Auch hier kann man durch Veränderung der Verweilzeit den Umsatz und damit die Selektivität gezielt einstellen.

Nach dem erfindungsgemäßen Verfahren erhält man alpha,omega-Aminonitrile in in guten Selektivitäten und mit nur geringen Mengen an Hexamethylendiamin. Des weiteren weisen die erfindungsgemäß eingesetzten Katalysatoren ein deutlich längere Standzeit auf als vergleichbare Katalysatoren aus dem Stand der Technik. Die alpha,omega-Aminonitrile sind wichtige Ausgangsverbindungen zur Herstellung von cyclischen Lactamen, insbesondere 6-Aminocapronitril für Caprolactam.

### Beispiele

### Vergleichsbeispiel 1: (Beispiel 2 in DE-A 848,654)

Ein Rohrreaktor von 4,5 m Länge und 0,6 cm Innendurchmesser wurde mit 105 ml (96 g) Katalysator, bestehend aus 2,3 Gew.-% PdO auf SiO₂ (Rest) befüllt und der Katalysator anschließend innerhalb von 48 h in einem Wasserstoffstrom (200 l/h) durch Erhöhung der Temperatur von 30°C auf 250°C drucklos aktiviert. Nach Absenkung der Temperatur auf 120°C wurde dem Reaktor bei 180 bar ein Gemisch aus 55 ml/h Adipodinitril (ADN), 130 ml/h Ammoniak und 200 l/h Wasserstoff zugeführt. Das Adipodinitril setzte sich unter diesen Bedingungen zu 13 % um. Das Reaktionsgemisch bestand im wesentlichen aus 87 Gew.-% ADN und 3,3 Gew.-% ACN (6-Amino-capronitril). Der Katalysator verlor unter diesen Bedingungen pro Betriebsstunde 3 % seiner Anfangsaktivität.

### Vergleichsbeispiel 2: (Beispiel 4 in DE-A 848,654)

Mit 4 Gew.-% CuO, 4 Gew.-% ZnO und 16,6 Gew.-% Co₂O₃ auf SiO₂ (Rest) als Katalysator wurde bei 80°C und 180 bar im gleichen Reaktor wie in Vergleichsbeispiel 1 ein Gemisch aus 55 ml/h Adipodinitril, 130 ml/h Ammoniak und 200 l/h Wasserstoff zu 50 % umgesetzt. Der Reaktionsaustrag bestand aus 50 Gew.-% ADN, 40 Gew.-% ACN und 9 Gew.-% HMD (Hexamethylendiamin). Durch Erhöhung der Reaktionstemperatur auf 95°C stieg der Umsatz auf 69 %. Das Reaktionsgemisch bestand im wesentlichen aus 31 Gew.-% ADN, 46 Gew.-% ACN und 21 Gew.-% HMD. Der Katalysator verlor unter diesen Bedingungen pro Betriebsstunde 1 % seiner Anfangsaktivität und die Formkörper waren nach 60 h vollständig zerfallen.

### Vergleichsbeispiel 3: (Beispiel 3 in DE-A 848,654)

Mit 7,5 Gew.-% CoO, 16 Gew.-% Fe₂O₃ auf SiO₂ (Rest) als Katalysator wurde bei 70°C und 180 bar im gleichen Reaktor wie in Vergleichsbeispiel 1 ein Gemisch aus 55 ml/h Adipodinitril, 130 ml/h Ammoniak und 200 l/h Wasserstoff zu 45 % umgesetzt. Der Reaktionsaustrag bestand im wesentlichen aus 55 Gew.-% ADN, 37 Gew.-% ACN und 7 Gew.-% HMD. Durch Erhöhung der Reaktionstemperatur auf 85°C stieg der Umsatz auf 78 %. Das Reaktionsgemisch bestand im wesentlichen aus 22 Gew.-% ADN, 48 Gew.-% ACN und 27 Gew.-% HMD. Der Katalysator verlor pro Betriebsstunde 0,5 % seiner Anfangsaktivität, innerhalb von 24 h 10 % seiner Anfangsaktivität.

### Beispiel 1:

Ein Rohrreaktor von 2 m Länge und 2,5 cm Innendurchmesser wurde mit 750 ml (1534 g) Katalysator, bestehend aus 90 Gew.-% CoO, 5 Gew.-% Mn₂O₃, 3 Gew.-% P₂O₅ und 2 Gew.-% Na₂O, befüllt, und der Katalysator wurde anschließend innerhalb von 48 h in einem Wasserstoffstrom (500 l/h) durch Erhöhung der Temperatur von 30°C auf 280°C drucklos aktiviert. Nach Absenkung der Temperatur auf 60°C wurde dem Reaktor bei 200 bar ein Gemisch aus 400 ml/h Adipodinitril, 930 ml/h Ammoniak und 500 l/h Wasserstoff zugeführt. Das Adipodinitril setzte sich unter diesen Bedingungen zu 46 % um. Das Reaktionsgemisch bestand im wesentlichen aus 54 Gew.-% ADN, 37 Gew.-% ACN und 9 Gew.-% HMD. Durch Erhöhung der Reaktionstemperatur auf 70°C stieg der Umsatz auf 65 %. Das Reaktionsgemisch bestand im wesentlichen aus 34,5 Gew.-% ADN, 46 Gew.-% ACN und 19,5 Gew.-% HMD. Der Katalysator zeigte nach 900 h bei unveränderter Aktivität noch die gleiche Selektivität wie Frischkatalysator. Die Formkörper des Katalysators waren nach dem Ausbau (nach 900 h) noch vollständig erhalten.

### Beispiel 2:

Ein Rohrreaktor von 4,5 m Länge und 0,6 cm Innendurchmesser wurde mit 105 ml (96 g) Katalysator, bestehend aus 20,0 Gew.-% NiO, 67,42 Gew.-% SiO₂, 3,7 Gew.-% Al₂O₃, 0,8 Gew.-% Fe₂O₃, 0,76 Gew.-% MgO, 1,92 Gew.-% CaO, 3,4 Gew.-% Na₂O, 2,0 Gew.-% K₂O, befüllt und der Katalysator wurde anschließend innerhalb von 48 h in einem Wasserstoffstrom (200 l/h) durch Erhöhung der Temperatur von 30°C auf 250°C drucklos aktiviert. Nach Absenkung der Temperatur auf 110°C wurde dem Reaktor bei 180 bar ein Gemisch aus 50 ml/h Adipodinitril, 130 ml/h Ammoniak und 200 l/h Wasserstoff zugeführt. Das Adipodinitril setzte sich unter diesen Bedingungen zu 25 % um. Das Reaktionsgemisch bestand im wesentlichen aus 75 Gew.-% ADN, 24 Gew.-% ACN und 0,5 Gew.-% HMD. Durch Erhöhung der Reaktionstemperatur auf 120°C stieg der Umsatz auf 60 %. Das Reaktionsgemisch bestand im wesentlichen aus 40 Gew.-% ADN, 53 Gew.-% ACN und 5 % HMD. Der Katalysator zeigte über 100 h eine konstante Aktivität.

### Beispiel 3:

### Langzeitversuch über 3000 h mit Katalysator wie Beispiel 1

Ein Rohrreaktor von 2 m Länge und 2,5 cm Innendurchmesser wurde mit 750 ml (1534 g) Katalysator, bestehend aus 90 Gew.-% Co 5 Gew.-% Mn₂O₃, 3 Gew.-% P₂O₅ und 2 Gew.-% Na₂O, befüllt, und der Katalysator wurde anschließend innerhalb von 48 h in einem Wasserstoffstrom (500 l/h) durch Erhöhung der Temperatur von 30°C auf 280°C drucklos aktiviert. Nach Absenkung der Temperatur auf 55°C (Eingang) bzw. 70°C (Ausgang) wurde dem Reaktor bei 200 bar ein Gemisch aus 400 ml/h Adipodinitril, 1900 ml/h Ammoniak und 500 l/h Wasserstoff zugeführt. Das Adiponitril setzt sich unter diesen Bedingungen zu 50 % um. Das Reaktionsgemisch bestand im wesentlichen aus 50 Gew.-% ADN, 39 Gew.-% ACN und 11 Gew.-% HMD (ACN-Selektivität: 78 %, ACN + HMD-Selektivität: 100 %). Der Katalysator zeigte nach 3000 h bei unveränderter Aktivität noch die gleiche Selektivität wie Frischkatalysator.

### Beispiel 4

### Katalysatorherstellung

Ein Fe-Katalysator wurde nach der in Catalyst Manufacture, A.B. Stiles, T.A. Koch (1995) S. 167/68 beschriebenen Methode durch Zusammenschmelzen von Fe-Oxid (Magnetit) mit den Promotoren Al₂O₃, K₂O als K₂CO₃ und CaO als Ca-Carbonat, danach Brechen und Sieben der erstarrten Schmelze erhalten. Der Katalysator hatte im oxidischen Zustand folgende Zusammensetzung: 1,1 Gew.-% K₂O, 3,0 Gew.-% Al₂O₃, 2,3 Gew.-% CaO, Rest FeO/Fe₂O₃.

(BET-Oberfläche: 6,5 m²/g, nach zehnstündiger Reduktion des Katalysators bei 450°C im H₂-Strom (drucklos) und anschließender Passivierung nach Abkühlung mit einem Luft-/Stickstoffgemisch).

### Versuchsergebnis

3 in Reihe geschaltete Rohrreaktoren (Gesamtlänge 4,5 m, d = 6 mm) wurden mit 115 ml (303 g) Katalysator befüllt und anschließend drucklos im Wasserstoffstrom (200 l/h) reduziert. Hierzu wurde die Temperatur innerhalb 24 h von 50°C auf 340°C angehoben und anschließend 72 h bei 340°C gehalten. Nach Absenken der Temperatur auf 120°C wurde dem Reaktor bei 250 bar ein Gemisch aus 55 ml/h ADN, 260 ml NH₃/h und 200 Nl Wasserstoff/h zugeführt.

Nach einer Anfahrphase von ca. 200 h wurde mit diesen Bedingungen ein ADN-Umsatz von 47 % erzielt. Das Reaktionsgemisch bestand im wesentlichen aus 53 Gew.-% ADN, 38 Gew.-% ACN und 8 % HMD (ACN-Selektivität: 80,9 %, ACN + HMD-Selektivität: 98 %). Ein solcher Reaktonsaustrag wurde über eine Laufzeit von 400 h erhalten.

### Beispiel 5

### Katalysatorherstellung

Durch Auflösen von Kobaltnitrat, Kupfernitrat, Mangannitrat und Phosphorsäure in Wasser wurde eine Lösung hergestellt, die 9,3 Gew.-% Kobalt, 2,7 Gew.-% Kupfer, 0,9 Gew.-% Mangan und 0,5 Gew.-% H₃PO₄ enthielt. Durch Zugabe einer 20%igen Natriumcarbonatlösung wurde bei einer Temperatur von 50°C gefällt. Der entstandene Niederschlag wurde gewaschen, bis im Waschwasser kein Natrium und Nitrat mehr nachweisbar waren. Der so erhaltene Feststoff wurde mit Wasser angemaischt und in einem Sprühturm versprüht (Eingangstemperatur = 550°C). Das Sprühgut wurde bei 500°C getrocknet, gekollert und im Extruder zu Strängen von 4 mm Durchmesser verformt. Die Stränge wurden bei 100 bis 120°C getrocknet und 1 h bei 900°C calciniert.

Die so erhaltenen Stränge wurden bei 320°C im Wasserstoffstrom reduziert und bei Raumtemperatur mit Stickstoff/Luft-Gemisch passiviert.

### Versuchsergebnisse

270 ml Autoklav (diskontinuierliche Versuche)
- Einsatz:: 36 g ADN, 54 g (89 ml) NH₃, 31 ml Katalysator (Formkörper)
- Katalysator:: 66 % CoO; 20 CuO; 7,3 % Mn₃O₄; 3,6 MoO₃; 0,1 % Na₂O; 3 % H₃PO₄ (53 g) (alle %-Angaben als Gew.-%)

Der Katalysator wurde vor Versuchsbeginn 10 h bei 200°C mit 20 l/h Wasserstoff behandelt.

Ergebnisse von Beispiel 5

| Temp./Druck [°C][bar] | Versuchsdauer [h] | ACN-Ausb. [%] | HMD-Ausb. [%] | ACN-Selekt. [%] | Umsatz [%] |
|---|---|---|---|---|---|
| 50/200 | 5 | 40 | 13 | 76 | 53 |
| 50/200 | 6 | 44 | 17 | 72 | 61 |

## Patentansprüche

1. Verfahren zur Herstellung von aliphatischen alpha,omega-Aminonitrilen durch partielle Hydrierung von aliphatischen alpha,omega-Dinitrilen bei erhöhter Temperatur und erhöhtem Druck in Gegenwart eines Lösungsmittels und eines Katalysators, dadurch gekennzeichnet, daß man einen Katalysator verwendet, der
(a) mindestens eine Verbindung auf der Basis eines Metalles, ausgewählt aus der Gruppe, bestehend aus Nickel, Cobalt, Eisen, Ruthenium und Rhodium, enthält und
(b) von 0,01 bis 25 Gew.-%, bezogen auf (a), mindestens eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut, Silicium, Titan, Zirkonium und Seltenerdmetalle, sowie
(c) von 0 bis 5 Gew.-%, bezogen auf (a), mindestens einer Verbindung auf der Basis eines Alkali- oder Erdalkalimetalles, enthält,
mit der Maßgabe, daß die Komponente (a) nicht auf der Basis von Eisen oder Eisen und einem der Metalle, ausgewählt aus der Gruppe, bestehend aus Cobalt, Ruthenium und Rhodium, besteht, wenn (b) ein Promotor auf der Basis eines Metalles, ausgewählt aus der Gruppe, bestehend aus Titan, Mangan, Chrom und Molybdän, ist sowie mit der weiteren Maßgabe, daß, wenn als Komponente (a) eine Verbindung auf der Basis von nur Ruthenium oder Ruthenium und Rhodium oder Nickel und Rhodium gewählt wird, der Promotor (b) gewünschtenfalls entfallen kann.

2. Verfahren nach Anspruch 1, dadurch gekennzeichnet, daß der Katalysator ein Trägerkatalysator ist.

3. Verfahren nach Anspruch 1, dadurch gekennzeichnet, daß der Katalysator ein Vollkatalysator ist.

4. Verfahren nach einem der Ansprüche 1 bis 3, dadurch gekennzeichnet, daß man die Hydrierung in einer Suspension vornimmt.

5. Verfahren nach einem der Ansprüche 1 bis 3, dadurch gekennzeichnet, daß man die Hydrierung in einem Festbettreaktor vornimmt.

6. Verfahren nach einem der Ansprüche 1 bis 5, dadurch gekennzeichnet, daß man als alpha,omega-Dinitril Adipodinitril einsetzt unter Erhalt von 6-Aminocapronitril.

7. Verfahren nach Anspruch 6, dadurch gekennzeichnet, daß man die Hydrierung bei einem Druck im Bereich von 2 bis 40 MPa durchführt.

8. Verfahren nach Anspruch 6 oder 7, dadurch gekennzeichnet, daß man die Hydrierung bei einer Temperatur im Bereich von 30 bis 150°C durchführt.

## Claims

1. A process for the preparation of aliphatic alpha,omega-amino-nitriles by partial hydrogenation of aliphatic alpha,omega-dinitriles at elevated temperatures and superatmospheric pressure in the presence of a solvent and of a catalyst, which comprises using a catalyst which
(a) contains at least one compound based on a metal selected from the group consisting of nickel, cobalt, iron, ruthenium and rhodium and
(b) contains from 0.01 to 25% by weight, based on (a), of at least one promoter based on a metal selected from the group consisting of palladium, platinum, iridium, osmium, copper, silver, gold, chromium, molybdenum, tungsten, manganese, rhenium, zinc, cadmium, lead, aluminum, tin, phosphorus, arsenic, antimony, bismuth, silicon, titanium, zirconium and rare earth metals and
(c) from 0 to 5% by weight, based on (a), of at least one compound based on an alkali metal or on an alkaline earth metal,
with the proviso that the component (a) is not based on iron or iron and one of the metals selected from the group consisting of cobalt, ruthenium and rhodium when (b) is a promoter based on a metal selected from the group consisting of titanium, manganese, chromium and molybdenum, and with the further proviso that, when a compound based on only ruthenium or ruthenium and rhodium or nickel and rhodium is selected as component (a), the promoter (b) may, if desired, be dispensed with.

2. A process as claimed in claim 1, wherein the catalyst is a supported catalyst.

3. A process as claimed in claim 1, wherein the catalyst is an unsupported catalyst.

4. A process as claimed in any of claims 1 to 3, wherein the hydrogenation is carried out in a suspension.

5. A process as claimed in any of claims 1 to 3, wherein the hydrogenation is carried out in a fixed-bed reactor.

6. A process as claimed in any of claims 1 to 5, wherein the alpha,omega-dinitrile used is adiponitrile, 6-aminocapro-nitrile being obtained.

7. A process as claimed in claim 6, wherein the hydrogenation is carried out at from 2 to 40 MPa.

8. A process as claimed in claim 6 or 7, wherein the hydrogenation is carried out at from 30 to 150°C.

## Revendications

1. Procédé de préparation d'aminonitriles alpha, oméga aliphatiques par hydrogénation partielle de dinitriles alpha, oméga aliphatiques, à température élevée et sous haute pression, en présence d'un solvant et d'un catalyseur, caractérisé en ce que l'on utilise un catalyseur contenant
(a) au moins un composé à base d'un métal choisi dans le groupe formé par le nickel, le cobalt, le fer, le ruthénium et le rhodium et
(b) de 0,01 à 25% en poids, par rapport à (a), d'au moins un promoteur à base d'un métal choisi dans le groupe formé par le palladium, le platine, l'iridium, l'osmium, le cuivre, l'argent, l'or, le chrome, le molybdène, le tungstène, le manganèse, le rhénium, le zinc, le cadmium, le plomb, l'aluminium, l'étain, le phosphore, l'arsenic, l'antimoine, le bismuth, le silicium, le titane, le zirconium et des terres rares, ainsi que
(c) de 0 à 5% en poids, par rapport à (a), d'au moins un composé à base d'un métal alcalin ou alcalino-terreux,
avec la condition que le composant (a) ne soit pas à base de fer ou de fer et de l'un des métaux choisis dans le groupe formé par le cobalt, le ruthénium et le rhodium, lorsque (b) est un promoteur à base d'un métal choisi dans le groupe formé par le titane, le manganèse, le chrome et le molybdène, et avec la condition supplémentaire que, lorsque l'on choisit comme composant (a) un composé uniquement à base de ruthénium ou de ruthénium et de rhodium ou de nickel et de rhodium, le promoteur (b) peut éventuellement être supprimé.

2. Procédé selon la revendication 1, caractérisé en ce que le catalyseur est un catalyseur supporté.

3. Procédé selon la revendication 1, caractérisé en ce que le catalyseur est un catalyseur plein.

4. Procédé selon l'une des revendications 1 à 3, caractérisé en ce que l'on entreprend l'hydrogénation partielle en suspension.

5. Procédé selon l'une des revendications 1 à 3, caractérisé en ce que l'on entreprend l'hydrogénation dans un réacteur à lit fixe.

6. Procédé selon l'une des revendications 1 à 5, caractérisé en ce que l'on met en oeuvre un nitrile adipique alpha,oméga-dinitrile, avec obtention de 6-aminocapronitrile.

7. Procédé selon la revendication 6, caractérisé en ce que l'on entreprend l'hydrogénation à une pression de 2 à 40 MPa.

8. Procédé selon la revendication 6 ou 7, caractérisé en ce que l'on entreprend l'hydrogénation à une température de 30 à 150°C.
